# EUROPÄISCHE PATENTSCHRIFT

(11) **EP 1 549 202 B1**
(45) Veröffentlichungstag und Bekanntmachung des Hinweises auf die Patenterteilung: **28.12.2005**
(21) Anmeldenummer: 04765505.5
(22) Anmeldetag: 22.09.2004
(51) Int. Cl.: A61B 1/00

(54) **ENDOSKOP FÜR MEDIZINISCHE UND NICHT-MEDIZINISCHE ZWECKE**
ENDOSCOPE FOR MEDICAL AND NON-MEDICAL PURPOSES
ENDOSCOPE POUR APPLICATION MEDICALE ET NON MEDICALE

(30) Priorität: 24.09.2003 DE 10344109
(43) Veröffentlichungstag der Anmeldung: 06.07.2005
(73) Patentinhaber: Karl Storz GmbH & Co. KG, 78532 Tuttlingen (DE)
(72) Erfinder: RENNER, Klaus, 78576 Emmingen-Liptingen (DE); SCHÜLE, Werner, 88637 Leibertingen (DE)
(74) Vertreter: Hofmeister, Frank
(86) Internationale Anmeldenummer: PCT/EP2004/010643
(87) Internationale Veröffentlichungsnummer: WO 2005/030043

(56) Entgegenhaltungen:
- DE-A- 10 113 365
- DE-A- 19 507 205
- DE-A- 19 647 851
- DE-U- 20 311 049

## Beschreibung

Die Erfindung betrifft ein Endoskop für medizinische und nicht-medizinische Zwecke, mit einem Gehäuse und einem in dem Gehäuse angeordneten, eine hygroskopische Substanz enthaltenden Formkörper. Weiterhin betrifft die Erfindung eine Trockenmittelanordnung für dieses medizinische Instrument.

Optische Instrumente und insbesondere Endoskope für medizinische und nichtmedizinische Zwecke sind prinzipiell fluiddichte Systeme. Durch eine Reihe von Gründen besteht aber die Möglichkeit, dass Feuchtigkeit in das Gehäuse eindringen kann, was zu einer die Sicht verschlechternden Trübung der optischen Systeme führen kann. Probleme können beispielsweise bereits bei der Fertigung der Instrumente in normaler Atmosphäre entstehen, wenn sich die Restfeuchte der Atmosphärenluft im Gehäuseinneren niederschlägt. Weiterhin kann Feuchtigkeit durch geringfügige Undichtigkeiten an Verbindungsstellen eindringen, an denen das Instrument zu Wartungs-, Reparatur- oder Montagezwecken auseinandernehmbar ist. Eine weitere starke Belastung der Instrumente stellt beispielsweise bei medizinischen Endoskopen das Reinigen mittels Autoklavieren dar, bei dem das Instrument unter wechselndem Druck Heißdampf bei etwa 140° C ausgesetzt wird. Diese Temperaturbelastung kann zu feinen Rissen führen, durch die wiederum Feuchtigkeit in das Gehäuse eindringen kann.

Zur Vermeidung solcher Beschlagprobleme durch sich auf dem optischen System niederschlagende Feuchtigkeit ist es bei optischen Instrumenten bekannt, im Gehäuse eine hygroskopische Substanz anzuordnen, die die im Gehäuseinnenraum anfallende Feuchtigkeit bindet, bevor diese sich auf dem mindestens einen optischen System niederschlägt.

So ist es beispielsweise bekannt, die hygroskopische Substanz in loser form im Gehäuse anzuordnen. Dies hat jedoch den Nachteil, dass beim Bewegen des Instruments Geräusche auftreten und zudem durch die Bewegung Abrieb der hygroskopischen Substanz erzeugt wird, der sich als Staub auf die optischen Systeme legen kann.

Aus der DE 101 13 365 A1 ist ein optisches Instrument bekannt. Bei diesem Instrument ist die hygroskopische Substanz in ein formbares Matrixmaterial eingebettet das auswechselbar in die Okularmuschel des Instruments einsetzbar ist. Das formbare Matrixmaterial dient dabei als Traggerüst für das hygroskopische Material.

Weiterhin wird In der DE 195 07 205 A1 vorgeschlagen, die hygroskopische Substanz unter einem abnehmbaren Wandstück des Gehäuses anzuordnen, das mit einer Kupplung gasdicht mit der übrigen Gehäusewand verbunden ist. Diese Ausführungsform ermöglicht zwar ein schnelles und einfaches Auswechseln der hygroskopischen Substanz, jedoch unter Inkaufnahme des Nachteils, dass das Endoskop eine weitere Öffnung aufweist, deren Fluiddichtigkeit gewährleistet werden muß.

Davon ausgehend liegt der Erfindung die **Aufgabe** zugrunde, ein Endoskop der eingangs genannten Art so auszugestalten, dass die hygroskopische Substanz bei größtmöglicher Trocknungswirkung einfach und sicher in das Gehäuse integrierbar ist. Weiterhin liegt der Erfindung die Aufgabe zugrunde, eine Trockenmittelanordnung zur Anordnung in einem derartigen medizinischen Instrument bereitzustellen.

Die erfindungsgemäße **Lösung** dieser Aufgabenstellung ist dadurch gekennzeichnet, dass auch das Bindemittel eine hygroskopisch wirksame Substanz ist.

Durch die erfindungsgemäße Verwendung einer hygroskopisch wirksame Substanz als Bindemittel zur Herstellung des aus dem Trockenmittel und dem Bindemittel geformten Formkörpers wird die hygroskopische Wirkung des eigentlichen Trockenmittels deutlich verstärkt.

Der solchermaßen ausgebildete Formkörper zeichnet sich somit dadurch aus, dass die von ihm ausgehende trocknende Wirkung nicht nur durch die eigentliche hygroskopische Substanz (beispielsweise Silika-Gel) bewirkt wird, sondern zusätzlich auch noch durch das zum Binden und Formen der hygroskopischen Substanz verwendete Bindemittel.

Gemäß einer praktischen Ausfühungsform der Erfindung wird vorgeschlagen, dass das hygroskopisch wirksame Bindemittel ein Tonmineral und insbesondere ein Trockenton ist. Beispielsweise bei der Verwendung von Bentonit als Bindemittel wird die hygroskopische Wirkung des aus der hygroskopischen Substanz und dem Bentonit geformten Formkörpers verstärkt, da Bentonit als quellfähiges Mehrschichtsilikat die Fähigkeit besitzt, große Wassermengen zu adsorbieren.

Durch die erfindungsgemäße Ausbildung des Formkörpers ausschließlich aus der mit einem Bindemittel versetzten hygroskopischen Substanz kann aufgrund des Verzichts auf ein Matrixmaterial als Traggerüst die Menge der hygroskopischen Substanz zur Ausbildung des Formkörpers erhöht werden, wodurch wiederum die Trocknungswirkung verbessert wird.

Gemäß einer bevorzugten Ausführungsform der Erfindung wird vorgeschlagen, dass das Mischungsverhältnis der hygroskopischen Substanz zum Bindemittel in der Ausgangsmischung, das heißt vor einer möglichen chemischen Reaktion der beiden Bestandteile miteinander, vorzugsweise in etwa 4:1 beträgt.

Zur Anordnung des aus der hygroskopischen Substanz gebildeten Formkörpers innerhalb des Instrumentengehäuses ist im Gehäuse eine Ausnehmung, beispielsweise in Form einer Nut, ausgebildet, so dass kein Bauraumverlust bei der Ausbildung des Instruments auftritt, der die sonstigen Eigenschaften des medizinischen Instruments beeinträchtigen könnte.

Gemäß einer praktischen Ausführungsform der Erfindung ist der Formkörper ringförmig ausgebildet und die zugehörige Aufnahme im Gehäuse als Ringnut ausgestaltet.

Weiterhin wird mit der Erfindung eine Trockenmittelanordnung zur Anordnung in einem Endoskop vorgeschlagen. Um sicherzustellen, dass keinerlei Partikel des Formkörpers in den Instrumenteninnenraum gelangen, umfasst diese Trockenmittelanordnung neben dem ausschließlich aus der mit einem Bindemittel versetzten hygroskopische Substanz bestehenden Formkörper ein mit dem Formkörper zusammenwirkendes Sieb.

Zur Ausbildung des wasserdampfdurchlässigen Siebs wird vorgeschlagen, dass dieses als feinmaschiges, vorzugsweise aus Metall bestehendes Sieb oder als permeable Membran ausgebildet ist.

Bei einer ersten Ausführungsform der Trockenmittelanordnung ist das Sieb als separates Bauteil auf der dem Gehäuseinneren zugewandten Seite des Formkörpers angeordnet. Alternativ wird mit der Erfindung weiterhin vorgeschlagen, dass das Sieb auf der dem Gehäuseinneren zugewandten Seite des Formkörpers in das Material des Formkörpers eingebettet ist wobei vorteilhafterweise der formkörper und das Sieb einen Rahmen bildend mit einem Kunststoffmaterial, insbesondere einem Duroplast, ummantelt sind

Schließlich wird mit der Erfindung vorgeschlagen, dass die den Formkörper bildenden hygroskopisch wirksamen Substanzen nach dem Zusammenbau und vordem ersten Einsatz des Endoskops durch eine entsprechende Temperaturbehandlung aktivierbar sind, um den Feuchtigkeitsgehalt des Trockenmittels zu minimieren und so dessen Trocknungseigenschaft zu erhöhen.

Weitere Merkmale und Vorteile der Erfindung ergeben sich anhand der nachfolgenden Beschreibung der zugehörigen Zeichnung, in der ein Ausführungsbeispiel eines erfindungsgemäßen Endoskops nur beispielhaft schematisch dargestellt ist. In der Zeichnung zeigt:
- Fig. 1a: einen Längsschnitt durch einen Teil eines erfindungsgemäßen Endoskops;
- Fig. 1b: eine vergrößerte Darstellung des Details gemäß Fig. 1a und
- Fig. 2: eine Explosionszeichnung des Instrumententeils gemäß Fig. 1a.

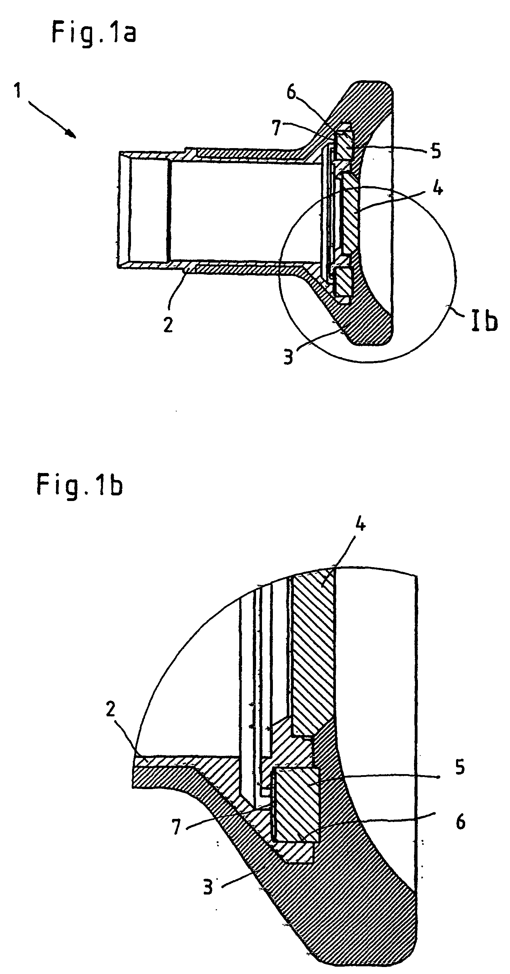

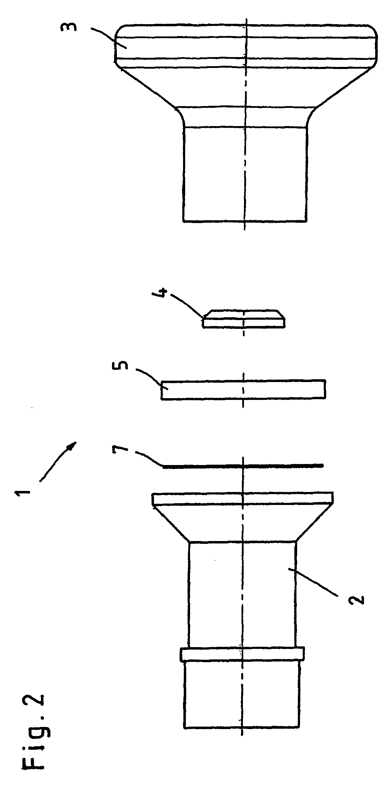

Bei dem in den Abbildungen Fig. 1a, 1b und 2 dargestellten Teil eines Instruments handelt es sich um eine Okulareinheit 1 eines Endoskops, wobei ein solches Endoskop sowohl für medizinische Zwecke als auch für nicht-medizinische Zwecke, beispielsweise technische Zwecke, verwendbar ist.

Die dargestellte Okulareinheit 1 besteht im wesentlichen aus einem Gehäuse 2, einer am proximalen Ende des Gehäuses 2 angeordneten Okularmuschel 3 sowie einem Okulardeckglas 4, welches beispielsweise in die Okutarmuschel 3 eingekittet, die Okulareinheit 1 proximalseitig fluiddicht verschließt. Distalseitig wird die Okulareinheit 1 mit dem Gehäuse des nicht dargestellten Endoskops verschraubt, wobei beim fertig montierten Endoskop, mindestens ein optisches System im Inneren der Okulareinheit 1 angeordnet ist.

Zum Entfernen der eventuell im Gehäuse 2 vorhandenen Restfeuchte sowie zur Aufnahme von über Undichtigkeiten in das Gehäuse 2 eingedrungener Feuchtigkeit ist im Inneren des Gehäuses 2 eine hygroskopische Substanz, wie Molekularsiebe, beispielsweise synthetische Zeolithe oder andere Tonminerale, oder auch Silika-Gel, angeordnet. Insbesondere die Verwendung der beispielsweise einen Porendurchmesser von 0,4 nm aufweisenden Molekularsiebe als hygroskopische Substanz ist vorteilhaft, da diese bei Temperaturen zwischen 250° und 300°C regenerierbar sind.

Bei ausreichend niedrigem Druck ist eine sehr gute Aktivierung des Trockenmittels, das heißt eine Reduzierung des Feuchtigkeitsgehalts auf unter 1%, auch schon bei einer Temperatur von etwa 200°C möglich. Für eine Totalaktivierung des Trockenmittels, das heißt einer Reduzierung des Feuchtigkeitsgehalts auf 0%, bedarf es in der Regel einer Temperatur von etwa 570°C.

Um einerseits eine größtmögliche Trocknungswirkung mittels der hygroskopischen Substanz zu erzielen, andererseits aber zu verhindern, dass beim Bewegen des Instruments Abrieb der hygroskopischen Substanz erzeugt wird, der sich als Staub im Gehäuseinneren und insbesondere auf die optischen Systeme niederlegen kann, ist die hygroskopische Substanz mit einem Bindmittel versetzt als ein in das Gehäuse 2 einsetzbarer Formkörper 5 ausgebildet. Als Bindemittel zum Festigen und Formbarmachen der hygroskopischen Substanz kann beispielsweise Bentonit oder eine andere ebenfalls hygroskopisch wirksame Substanz verwendet werden, wobei das Mischungsverhältnis der hygroskopischen Substanz zum Bindemittel in der Ausgangsmischung, also vor einer möglichen chemischen Reaktion der Bestandteile miteinander, vorzugsweise in etwa 4:1 beträgt.

Vorteilhafterweise erfolgt eine erste Voraktivierung der den Formkörper bildenden hygroskopisch wirksamen Substanzen bereits nach dem Zusammenbau und vor dem ersten Einsatz des Endoskops, um den Feuchtigkeitsgehalt des Trockenmittels zu minimieren und so dessen Trocknungseigenschaft zu erhöhen.

Bei dem in den Abbildungen dargestellten Ausführungsbeispiel ist der Formkörper 5 als in eine umlaufende Ringnut 6 im Gehäuse 2 einsetzbarer Ring ausgebildet. Die Anordnung des Formkörpers 5 in der Ringnut 6 hat den Vorteil, dass die Anordnung des Trockenmittels keinen Bauraumverlust bewirkt, da die im Gehäuse 2 ausgebildete Ringnut 6 den freien Durchmesser des Gehäuseinneren nicht verringert.

Neben dieser Ausgestaltungsform des Formkörpers 5 als Ring sind selbstverständlich auch andere Ausgestaltungen, wie beispielsweise in Kugelform, möglich, wobei bei dieser Ausgestaltungsform die kugeligen Formkörper 5 zur Lagefixierung in einem Käfig anzuordnen sind.

Wie weiterhin aus den Abbildungen ersichtlich, umfasst die Trockenmittelanordnung bei dem dargestellten Ausführungsbeispiel zusätzlich ein wasserdampfdurchlässiges Sieb 7, dass verhindern soll, dass Partikel des Formkörper 5 in das Innere des Gehäuses 2 gelangen können. Bei der dargestellten Ausführungsform ist das Sieb 7 als separates Bauteil ausgebildet auf der dem Gehäuseinneren zugewandten Seite des Formkörpers 5 angeordnet. Alternativ zu dieser Ausgestaltung besteht die Möglichkeit, das Sieb 7 auf der dem Gehäuseinneren zugewandten Seite des Formkörpers 5 in das Material des Formkörpers 5 einzubetten und so eine Trockenmitteleinheit zu bilden.

Das Sieb 7 kann beispielsweise als feinmaschiges, vorzugsweise aus Metall bestehendes Sieb oder als permeable Membran ausgebildet sein.

Um die Trockenmittelanordnung aus Formkörper 5 und Sieb 7 einfach und betriebssicher in die entsprechende Ausnehmung im Gehäuse 2 einsetzen zu können, werden der Formkörper 5 und das Sieb 7 einen Rahmen bildend mit einem Kunststoffmaterial, insbesondere einem Duroplast, umspritzt oder anderweitig ummantelt, wie beispielsweise überzogen, umgossen oder umformt.

Durch das Herstellen des in das Gehäuse 2 einsetzbaren Formkörpers 5-ohne tragende Matrixstruktur ausschließlich aus der hygroskopischen Substanz und dem Bindemittel besteht der Formkörper 5 überwiegend aus der hygroskopischen Substanz, so dass eine größtmögliche Menge der hygroskopischen Substanz zu Trocknungszwecken zur Verfügung steht.

Darüber hinaus zeichnet sich die Anordnung dadurch aus, dass eine solche Trockenmittelanordnung zu Montage- und Wartungszwecken einfach und schnell ein und wieder ausbaubar ist.

### Bezugszeichenliste

- 1: Okulareinheit
- 2: Gehäuse
- 3: Okularmuschel
- 4: Okulardeckglas
- 5: Formkörper
- 6: Ringnut
- 7: Sieb

## Patentansprüche

1. Endoskop für medizinische und nicht-medizinische Zwecke, mit einem Gehäuse (2) und einem in dem Gehäuse (2) angeordneten, eine hygroskopische Substanz enthaltenden Formkörper (5), wobei der Formkörper (5) ausschließlich aus der mit einem Bindemittel versetzten hygroskopischen Substanz besteht,
**dadurch gekennzeichnet,**
**dass** auch das Bindemittel eine hygroskopisch wirksame Substanz ist.

2. Endoskop nach Anspruch 1, **dadurch gekennzeichnet, dass** das Bindemittel ein Tonmineral, insbesondere ein Trockenton, ist.

3. Endoskop nach Anspruch 2, **dadurch gekennzeichnet, dass** das Bindemittel Bentonit ist.

4. Endoskop nach einem der Ansprüche 1 bis 3, **dadurch gekennzeichnet, dass** das Mischungsverhältnis hygroskopische Substanz zu Bindemittel in der Ausgangsmischung in etwa 4:1 beträgt.

5. Endoskop nach einem der Ansprüche 1 bis 4, **dadurch gekennzeichnet, dass** im Gehäuse (2) eine Ausnehmung zur Aufnahme des Formkörpers (5) ausgebildet ist.

6. Endoskop nach Anspruch 5, **dadurch gekennzeichnet, dass** der Formkörper (5) ringförmig ausgebildet ist und die Ausnehmung im Gehäuse (2) als Ringnut (6) ausgestaltet ist.

7. Trockenmittelanordnung zur Anordnung in einem Endoskop nach einem der Ansprüche 1 bis 6,
**gekennzeichnet durch**
den ausschließlich aus der mit dem hygroskopisch wirksamen Bindemittel versetzten hygroskopischen Substanz bestehenden Formkörper (5) und einem mit dem Formkörper (5) zusammenwirkenden wasserdampfdurchlässigen Sieb (7).

8. Trockenmittelanordnung nach Anspruch 7 **dadurch gekennzeichnet, dass** das Sieb (7) als feinmaschiges Metallsieb ausgebildet ist.

9. Trockenmittelanordnung nach Anspruch 7 oder 8, **dadurch gekennzeichnet, dass** das Sieb (7) auf der dem Gehäuseinneren zugewandten Seite des Formkörpers (5) angeordnet ist.

10. Trockenmittelanordnung nach einem der Ansprüche 7 bis 9, **dadurch gekennzeichnet, dass** das Sieb (7) auf der dem Gehäuseinneren zugewandten Seite des Formkörpers (5) in das Material des Formkörpers (5) eingebettet ist.

11. Trockenmittelanordnung nach einem der Ansprüche 7 bis 10, **dadurch gekennzeichnet, dass** der Formkörper (5) und das Sieb (7) einen Rahmen bildend mit einem Kunststoffmaterial, insbesondere einem Duroplast, ummantelt sind.

12. Trockenmittelanordnung nach einem der Ansprüche 7 bis 11, **dadurch gekennzeichnet, dass** die den Formkörper (5) bildenden hygroskopisch wirksamen Substanzen nach dem Zusammenbau des Endoskops durch eine entsprechende Temperaturbehandlung aktivierbar sind.

## Claims

1. Endoscope for medical and non-medical purposes, with a housing (2) and with a moulded body (5) which is arranged in the housing (2) and contains a hygroscopic substance, the moulded body (5) being composed exclusively of the hygroscopic substance with an added binding agent, **characterized in that** the binding agent is also a hygroscopically active substance.

2. Endoscope according to Claim 1, **characterized in that** the binding agent is a clay mineral, in particular a dry clay.

3. Endoscope according to Claim 2, **characterized in that** the binding agent is bentonite.

4. Endoscope according to one of Claims 1 to 3, **characterized in that** the mixing ratio of hygroscopic substance to binding agent in the starting mix is approximately 4:1.

5. Endoscope according to one of Claims 1 to 4, **characterized in that** a recess for receiving the moulded body (5) is formed in the housing (2).

6. Endoscope according to Claim 5, **characterized in that** the moulded body (5) has an annular shape and the recess in the housing (2) is configured as an annular groove (6).

7. Desiccant unit for arrangement in an endoscope according to one of Claims 1 to 6, **characterized by** the moulded body (5) composed exclusively of the hygroscopic substance with the added hygrosopically active binding agent, and by a sieve (7) which is permeable to water vapour and interacts with the moulded body (5).

8. Desiccant unit according to Claim 7, **characterized in that** the sieve (7) is designed as a fine-meshed metal sieve.

9. Desiccant unit according to Claim 7 or 8, **characterized in that** the sieve (7) is arranged on that side of the moulded body (5) facing towards the inside of the housing.

10. Desiccant unit according to one of Claims 7 to 9, **characterized in that**, on that side of the moulded body (5) facing towards the inside of the housing, the sieve (7) is embedded in the material of the moulded body (5).

11. Desiccant unit according to one of Claims 7 to 10, **characterized in that** the moulded body (5) and the sieve (7) are surrounded by a plastic material, in particular a thermosetting material, forming a frame.

12. Desiccant unit according to one of Claims 7 to 11, **characterized in that**, after the endoscope has been assembled, the hygroscopically active substances forming the moulded body (5) can be activated by suitable temperature treatment.

## Revendications

1. Endoscope à usage médical et non médical avec un boîtier (2) et une pièce façonnée (5) contenant une substance hygroscopique agencée dans le boîtier (2), où la pièce façonnée (5) consiste exclusivement en la substance hygroscopique additionnée d'un liant, **caractérisé en ce que** le liant est aussi une substance efficace du point de vue hygroscopique.

2. Endoscope selon la revendication 1 **caractérisé en ce que** le liant est un minéral argileux, en particulier une argile sèche.

3. Endoscope selon la revendication 2 **caractérisé en ce que** le liant est la bentonite.

4. Endoscope selon l'une des revendications 1 à 3 **caractérisé en ce que** le rapport de mélange substance hygroscopique à liant dans le mélange de départ est d'environ 4 : 1.

5. Endoscope selon l'une des revendications 1 à 4 **caractérisé en ce qu'**un évidement est formé dans le boîtier (2) pour recevoir la pièce façonnée (5).

6. Endoscope selon la revendication 5 **caractérisé en ce que** la pièce façonnée (5) est annulaire et l'évidement dans le boîtier (2) est sous forme de rainure annulaire (6).

7. Agencement d'agent desséchant destiné à être agencé dans un endoscope selon l'une des revendications 1 à 6 **caractérisé par** la pièce façonnée (5) consistant exclusivement en la substance hygroscopique additionnée du liant efficace du point de vue hygroscopique et un tamis perméable à la vapeur d'eau (7) coopérant avec la pièce façonnée (5).

8. Agencement d'agent desséchant selon la revendication 7 **caractérisé en ce que** le tamis (7) est sous forme de tamis métallique à mailles fines.

9. Agencement d'agent desséchant selon la revendication 7 ou 8 **caractérisé en ce que** le tamis (7) est agencé sur le côté de la pièce façonnée (5) tourné vers l'intérieur du boîtier.

10. Agencement d'agent desséchant selon l'une des revendications 7 à 9 **caractérisé en ce que** le tamis (7) est inclus dans la matière de la pièce façonnée (5) du côté de la pièce façonnée (5) tourné vers l'intérieur du boîtier.

11. Agencement d'agent desséchant selon l'une des revendications 7 à 10 **caractérisé en ce que** la pièce façonnée (5) et le tamis (7) sont entourés par une matière plastique, en particulier une matière thermodurcissable, en formant un cadre.

12. Agencement d'agent desséchant selon l'une des revendications 7 à 11 **caractérisé en ce que** les substances efficaces du point de vue hygroscopique formant la pièce façonnée (5) sont activables par un traitement thermique correspondant après l'assemblage de l'endoscope.
